# EUROPEAN PATENT SPECIFICATION

(11) **EP 3 846 743 B1**
(45) Date of publication and mention of the grant of the patent: **04.11.2026**
(21) Application number: 19773636.6
(22) Date of filing: 09.09.2019
(51) Int. Cl.: A61F 2/40

(54) **UNIVERSAL SHOULDER PROSTHESIS SYSTEM**
UNIVERSELLES SCHULTERPROTHESENSYSTEM
SYSTÈME UNIVERSEL DE PROTHÈSE D'ÉPAULE

(30) Priority: 07.09.2018 US 201862728394 P; 12.06.2019 US 201916439639
(43) Date of publication of application: 14.07.2021
(73) Proprietor: Convertatek Shoulder Prosthesis Solutions, LLC, Old Tappan, NJ 07657 (US)
(72) Inventor: LONGOBARDI, Raphael S.F., Old Tappan, NJ 07675 (US)
(74) Representative: Viering, Jentschura & Partner mbB Patent- und Rechtsanwälte
(86) International application number: PCT/US2019/050253
(87) International publication number: WO 2020/051595

(56) References cited:
- DE-A1- 102006 041 550
- US-A1- 2008 294 268
- US-B1- 6 783 549

## Description

### I. Field of the Invention

The invention relates to a modular shoulder prosthesis system that provides for flexibility in shoulder replacements and allows for a more efficient switch for a patient between a traditional anatomic Total Shoulder Replacement (ta-TSR) to a reverse Total Shoulder Replacement (r-TSR). In at least one embodiment, the system also, or alternatively, provides for a modular adaptation for the glenoid side in a Total Shoulder Replacement (TSR).

### II. Background of the Invention

TSRs have evolved over the last 70 years, with the greatest degree of its evolution occurring within the past 20 years. The understanding of the complexity of the shoulder has resulted in the ability to better treat the multiple conditions that afflict the shoulder. Glenohumeral arthritis ranges from simple to complex due to etiology and deformity. Post traumatic glenohumeral arthritis, along with the deformity of both the glenoid and humeral head present challenges for the shoulder arthroplasty surgeon. Similarly, the problem of rotator cuff deficiency and rotator cuff arthropathy has resulted in the development of treatment and prosthetic design specific to address the loss of the main motors of the shoulder.

Currently, there are two types of TSR - traditional anatomic total shoulder replacement (ta-TSR) and reverse total shoulder replacement (r-TSR). Ta-TSR utilizes resurfacing of the humeral head and glenoid in the setting of an intact and functioning rotator cuff. Glenohumeral arthritis has been treated with ta-TSR, the current gold standard being the resurfacing of the humeral head with a stemmed or metaphyseal component along with a replacement of the humeral head articular portion with a Cobalt-Chromium (Co-Cr) implant. Modularity of the humeral components allows for appropriate sizing of the head in diameter and thickness to match the resected articular surface of the patient.

To revise from the ta-TSR to r-TSR often requires removal of the glenoid component and reconstruction of the glenoid bone stock. Ta-TSR utilize all polyethylene glenoid components which become the typical point of failure for the ta-TSR. R-TSR utilizes a porous in-growth metal design with locking screws for glenoid fixation. Modularity has always centered on being able to change the humeral components from ta-TSR to r-TSR, for example, a humeral head component to a socket configuration.

The resurfacing of the glenoid has evolved over the past 70 years. Originally, polyethylene bonded to metal, also known as metal-backed glenoids, was cemented into the glenoid bone. These failed at the polyethylene-metal interface due to stresses and edge loading of the component. What evolved was the use of all polyethylene components. First, all polyethylene with a keel was used, followed by all polyethylene with multiple pegs.

There was a higher rate of failure for the cemented keeled components, so currently the gold standard for glenoid resurfacing in ta-TSR is a cemented pegged, all polyethylene component.

The most common cause of failure of the ta-TSR is due to glenoid loosening secondary to rotator cuff failure/tear. The resulting superior migration of the humeral head, with concomitant change in the center of rotation (C.O.R.) from rotator cuff failure produces edge loading of the glenoid component. This asymmetric mechanical loading results in rocking and loosening of the polyethylene prosthesis from the cement and bone of the glenoid.

R-TSR evolved from the specific abnormal mechanics of the rotator cuff deficient shoulder, as previously described. In the rotator deficient condition, the deltoid muscle becomes the predominant motor, but in an inefficient manner. The deltoid muscle contraction functions to result in "hinged abduction" of the humeral head/humeral shaft. The humeral head and greater tuberosity lever on the undersurface of the acromion and superior portion of the glenoid. Ta-TSR is contra-indicated in the setting of rotator cuff deficiency, due to the known catastrophic results to the glenoid component.

The development of the r-TSR addresses the rotator cuff deficient, painful arthritic shoulder. The design of r-TSR is to maximize deltoid fiber length to allow more efficient contraction and function of the deltoid in elevation of the arm. The prosthetic components are designed to change the C.O.R. to one that is more inferior and medial to the native joint.

The design of r-TSR has also evolved over the past 20 years. The original "Grammont" style sought to inferiorly displace the humerus to maximize deltoid fiber length; this resulted in inferior scapular notching, leading to failure. The current revised designs include a C.O.R. which is more lateral and inferior to the native C.O.R. The implant design is for an in-growth trabecular metal baseplate with locking screws to secure the component to the bony glenoid. The relatively minimally curved glenoid is replaced with a glenosphere: a solid Co-Cr semi-spherical to ¾ spherical surface that attaches to the in-growth metal base-plate. This is typically through a combination of a Morse taper fit and center screw fixation. The glenosphere is typically inserted at an inferiorly directed version angle, between 5-10 degrees. This allows for inferior offset of the humerus, elongation of the deltoid muscle fibers and a joint reactive force in line with prosthetic alignment.

R-TSR already have asymmetric, higher shear and higher loading of the glenoid component, called the glenosphere and baseplate construct. Despite these greater loads, in growth metal baseplates with locking screws are not the cause of failure, due to the excellent bone incorporation and stability.

The r-TSR has a different humeral component design as well. Where the ta-TSR has the Co-Cr humeral head, the r-TSR had the Co-Cr glenosphere attached to the glenoid. The humerus had a stemmed component but attached to the top is a polyethylene cup or humeral cup to articulate with the glenosphere. The modularity of components, specifically glenosphere sizing and humeral cup sizing, allow for multiple permutations to achieve the most successful and stable construct.

Current long-term studies on viability of r-TSR have revealed that the construct of an in-growth metal baseplate with locking screws has excellent long-term fixation without evidence of loosening, even in osteoporotic bone.

Currently, there is not a single company known to the inventor that has a universal baseplate for both ta-TSR and r-TSR. Further, the modularity that exists today allows only for conversion of a ta-TSR humeral stem to accommodate a r-TSR humeral cup. Conversion to a r-TSR requires removal of the polyethylene glenoid with reconstruction of the glenoid bone with tricortical bone, often taken from the patient's own iliac crest, and consequential donor site morbidity. A modular shoulder prosthesis system according to the preamble of claim 1 is, e.g., known from US 2008/294268 A1.

### III. Summary of the Invention

In at least one embodiment, a modular system will allow the surgeon to achieve either exchanges, humeral or glenoid component, without extravagant amount of equipment to be processed and secured, or more complex operative procedures to be performed. A truly versatile and modular system would allow for a baseplate to accept either a traditional anatomic glenoid component or a reverse total shoulder glenosphere, without compromising long term security and function. At least one embodiment according to the invention will allow for all of this.

The novelty of this design, in at least one embodiment, allows for a single glenoid component that can be used for traditional, anatomic TSR, primary reverse TSR and revision of anatomic to reverse TSR. In at least one embodiment, a porous metal baseplate with locking screw fixation is used for the traditional anatomic glenoid component. An all metal glenoid component composed of the typical Cobalt-Chromium (Co-Cr), which is the most current and common alloy used in joint replacement, is press-fit onto the baseplate utilizing, for example, a Morse taper central fixation peg. The smooth, Co-Cr glenoid component, in at least one embodiment, would have anterior sided flanges to assist with the removal of the component. In at least one further embodiment, the flanges will provide additional connection points between the base and the modular component.

By using the same concept for securing the ta-TSR glenoid components, this prosthetic design will diminish glenoid loosening as a reason for revision of a ta-TSR. Further, the all metal glenoid baseplate solves the issue of glenoid bone loss and glenoid deformity during primary ta-TSR. In cases of posterior glenoid bone loss, the B2 or C glenoid types, the in-growth metal baseplates can be pre-fashioned/constructed to include posterior wedges: neutral, 10 degree and 25-degree wedges to allow for complete reconstruction of the glenoid using ingrowth trabecular metal and locking screws. Regardless of glenoid bony deformity and defects, a neutral or wedge baseplate can be used for either the ta-TSR or the r-TSR in the primary condition.

If the rotator cuff failure occurs and necessitates conversion to a r-TSR, the revision is facilitated by the modularity of the already inserted baseplate and the humeral base (for example, a humeral stem or a second baseplate) with the different modular components. First, the dual mobility humeral head can be removed from the humeral base. As long as the humeral fixation of the humeral base is intact, a modular humeral cup can be secured to the humeral base as part of the conversion to a r-TSR. Second, the Co-Cr glenoid surface can be removed from the baseplate and the glenosphere can be attached without revising the baseplate. In at least one embodiment, built into the glenosphere component will be a 7 or 10-degree inferior inclination, to allow for proper tensioning of the deltoid to maximize its function.

The invention provides a modular shoulder prosthesis system according to claim 1. Further embodiments are described in the dependent claims.

### IV. Brief Description of the Drawings

Any cross-hatching present in the figures is not intended to identify or limit the type of material present for the element shown in cross-section. In figures that include multiple elements shown in cross-section, the cross-hatching will be different directions for the different elements.
FIGs. 1A-1C illustrate a baseplate according to at least one embodiment of the invention. FIG. 1A illustrates a top view. FIG. 1B illustrates a side view with phantom lines illustrating the internal construction according to at least one embodiment of the invention. FIG. 1C illustrates an alternative cross-section of the baseplate for an alternative attachment with a glenosphere modular component from a view from superiorly of the glenosphere.
FIGs. 1D and 1E illustrate side views of alternative baseplates according to at least two embodiments of the invention.
FIGs. 2A and 2B illustrate a modular glenoid component according to at least one embodiment of the invention. FIG. 2B is a cross-section taken at 2B-2B in FIG. 2A, which illustrates a top view of the modular glenoid component.
FIG. 2C illustrates a top view of an alternative modular glenoid component according to at least one embodiment of the invention.
FIG. 2D illustrates a side view of another alternative modular glenoid component according to at least one embodiment of the invention.
FIGs. 3A-3D illustrate a glenosphere component according to at least two embodiments of the invention. FIG. 3A illustrates a side view. FIG. 3B is a cross-section taken along a vertical plane taken at 3B, 3C-3B, 3C in FIG. 3D, which illustrates a face-on view or view from lateral shoulder view. FIG. 3C illustrates an alternative glenosphere component as a cross-section taken at 3B, 3C-3B, 3C in FIG. 3D.
FIG. 3E illustrates a face-on view or view from lateral shoulder view of an alternative glenosphere component according to at least one embodiment of the invention.
FIG. 3F illustrates a top view of another alternative glenosphere component according to at least one embodiment of the invention.
FIG. 4A illustrates a side view of a humeral head according to at least one embodiment of the invention. FIG. 4B illustrates a cross-section of the humeral head illustrated in FIG. 4A taken along a vertical plane taken through a diameter of the humeral head.
FIGs. 5A and 5B illustrate cross-sections of examples of a modular glenoid component and humeral head implanted in bone according to at least two embodiments of the invention.
FIG. 6A illustrates a side view of a humeral cup according to at least one embodiment of the invention. FIG. 6B illustrates a cross-section of the humeral cup illustrated in FIG. 6A taken along a vertical plane taken through a diameter of the humeral cup with a shell insert according to at least one embodiment of the invention.
FIGs. 7A-7C illustrate an alternative humeral cup according to at least one embodiment of the invention. FIG. 7A illustrates a top view. FIG. 7B illustrates a cross-section taken at 7B-7B in FIG. 7A. FIG. 7C illustrates a side view of an alternative humeral cup according to at least one embodiment of the invention.
FIGs. 8A and 8B illustrate cross-sections of examples of a baseplate being used in place of a humeral stem according to at least two embodiments of the invention.

### V. Detailed Description of the Drawings

The invention in at least one embodiment includes a modular shoulder replacement system having a glenoid baseplate configured for attachment to a patient's glenoid with at least one attachment mechanism. Examples of attachment mechanism include screws, variable angle locking screws, and an impactable central cylinder. The baseplate has a mounting surface for engagement of a modular glenoid component for a ta-TSR or a modular glenosphere component for a r-TSR. The mounting surface refers to the substantially planar surface of the baseplate opposite the glenoid. Both the modular glenoid component and the modular glenosphere component are examples of a modular component. The attachment in at least one embodiment between the modular component and the baseplate is through, for example, a Morse taper, which may be a dual threaded Morse taper that is axially located with reference to the baseplate. In an alternative embodiment with or without the Morse taper, a torque limiting fastener, such as a screw or a bolt, is used to further secure the modular glenoid component to the baseplate by engaging the baseplate and/or the central attachment mechanism anchored in the patient's glenoid. In a further embodiment, the attachment is facilitated with a threaded connection where the modular component is screwed into the baseplate.

In at least one embodiment the modular shoulder replacement system further includes a humeral stem for attachment to a patient's humeral bone. The humeral stem having a receiving socket for insertion of a post (or other connection piece) from a modular humeral head for a ta-TSR or a modular humeral cup for a r-TSR. Both the modular humeral head and the modular humeral cup are examples of a modular humeral component. In at least one embodiment, the modular humeral component is attached to the humeral stem using, for example, a Morse taper, which may be a dual threaded Morse taper. In an alternative embodiment, the system includes a mounting base that is attached to the humeral stem component on to which the modular humeral head or modular humeral cup is attached. In an alternative embodiment with or without the Morse taper, a torque limiting fastener, such as a screw or a bolt, is used to further secure the modular humeral component to the humeral stem by engaging the receiving socket. In a further embodiment, the attachment is facilitated with a threaded connection where the modular humeral component is screwed into the humeral stem.

In an alternative embodiment, a baseplate used for the glenoid is adapted for use instead with the humerus in place of the humeral stem. In at least one embodiment, the glenoid baseplate (e.g., a first baseplate) and the humeral baseplate (e.g., a second baseplate) are identical. In at least one alternative embodiment, the humeral baseplate is larger than the glenoid baseplate. In a further embodiment, the humeral baseplate has a larger diameter (e.g., for the base and/or the stem), height, and/or thickness than the glenoid baseplate. Examples of diameters for the baseplates include about 25 mm, 29 mm, 33 mm, 37 mm, 41 mm, a range between 25 mm and 41 mm, or a range of 25 mm to 33 mm where the measurements in this disclosure also include approximations given manufacturing tolerances.

The baseplate and the humeral stem (or a second baseplate) are designed to remain in place while switching the modular component and the modular humeral component, respectively, to switch from a ta-TSR to a r-TSR.

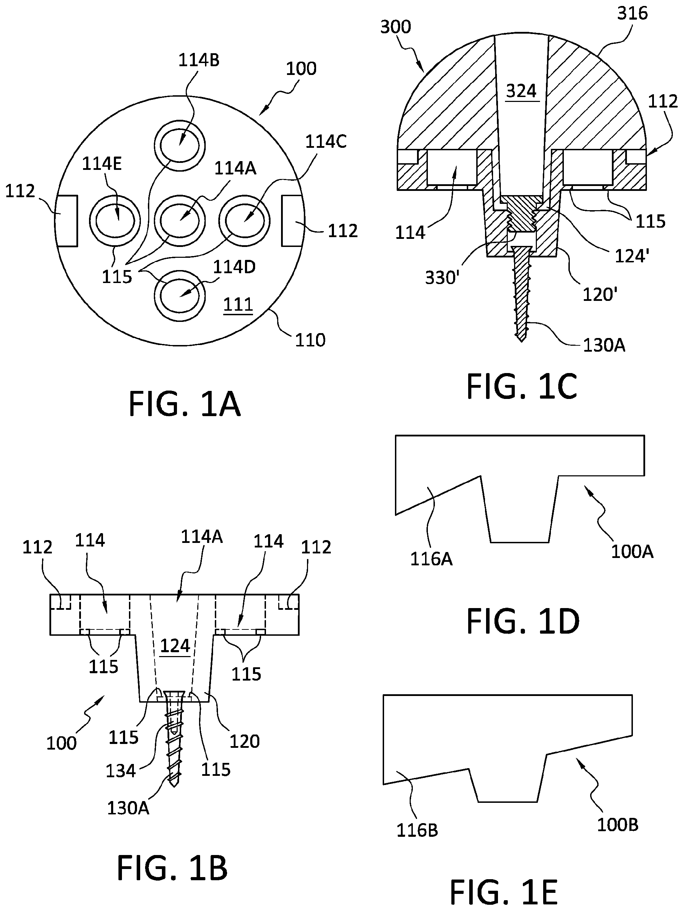

FIGs. 1A and 1B illustrate a baseplate 100 according to at least one embodiment. The illustrated baseplate 100 includes a base 110 and a stem 120 extending from the base 110. Examples of diameters for the base 110 include between 25 mm and 30 mm, in a further example including the end points of that range, 25 mm, or 29 mm. Although the base 110 is illustrated as being circular, the base 110 may be elliptical, oval, or other suitable shapes; in such an embodiment, the modular component may be shaped to match. Examples of the thickness of the base 110 include between 5 mm and 15 mm (with or without the end points), 5 mm, 7 mm, 10 mm, 12.5 mm, and 15 mm. In at least one embodiment, the baseplate 100 is made from an ingrowth trabecular metal over a metal core of, for example, steel, titanium or a combination. The ingrowth trabecular metal facilitates bone ingrowth into the baseplate 100, for example to increase the strength of the connection between the bone and the baseplate 100 and the respective interface shear strength over time.

Although the stem 120 in FIG. 1B is illustrated as a cylinder with a slight taper, in at least one embodiment, the stem 120 has tapered sides to match the modular component plugs. In a further embodiment, the stem 120 is substantially cylindrical.

The base 110 includes a mounting surface 111 on a side opposite of the side from which the stem 120 extends. In at least one embodiment, the mounting surface 111 is substantially planar. The plane defined by the mounting surface 111 is approximately parallel to the glenoid resection plane after implantation. In an alternative embodiment, the plane defined by the mounting surface 111 is at an angle to the glenoid resection plane after implantation. The mounting surface 111 in at least one embodiment will be a sufficient height above the glenoid resection plane to allow access to notches 112, 112.

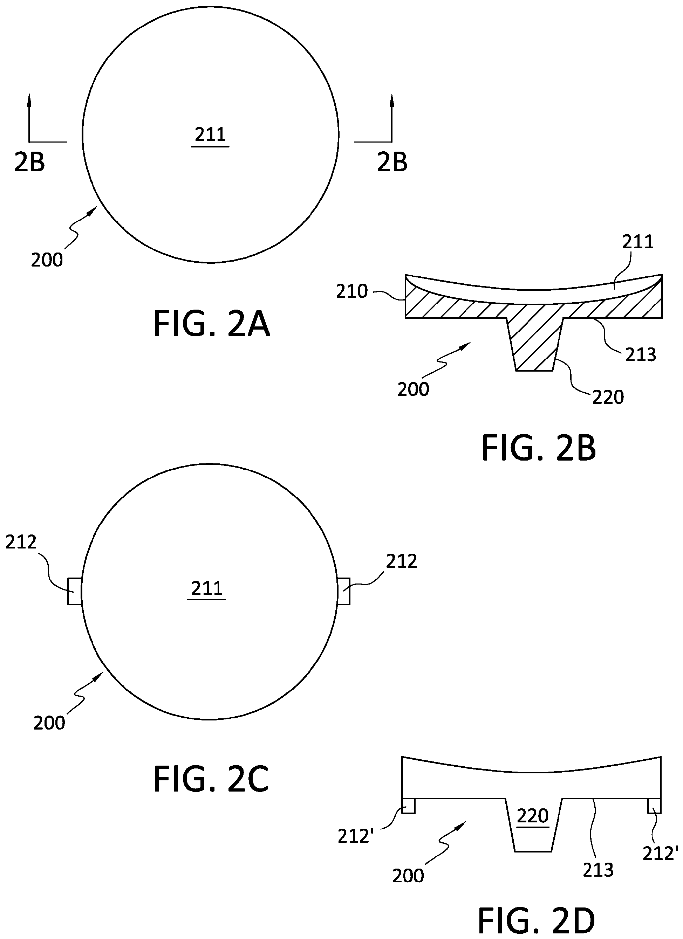

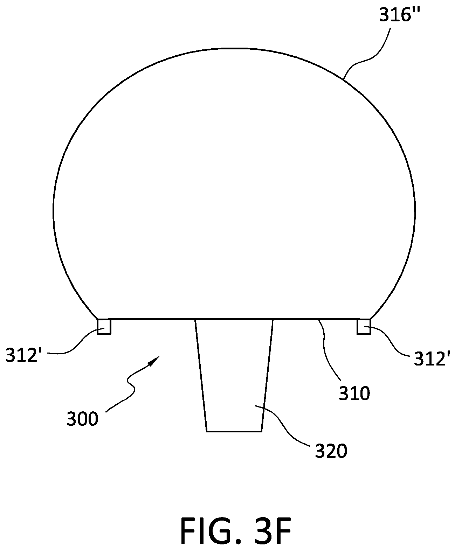

The illustrated base 110 includes a pair of opposed notches 112, 112 extending down from outer circumferential sides of the mounting surface 111, for example on the anterior and posterior central edge (or side) of the base 110, which in at least one embodiment provides better access to the notches for removal of the modular component. The notches 112, 112 are configured to be accessible from the mounting surface 111. In at least one embodiment, the notches 112, 112 provide (or are configured to have) a leverage point to facilitate separation of the mounted modular component from the baseplate 100 when the baseplate 100 is affixed to the glenoid. The notches 112, 112 have sufficient width and depth to receive an instrument in which to pry the mounted modular component from the baseplate 100. Although two notches 112, 112 are illustrated, additional notches could be added to the base 110. In a further embodiment, each of the notches 112, 112 receive a flange 212'/312', 212'/312' (*see*, *e.g*., FIGs. 2D and 3F) depending from a base of the modular component, for example to increase the strength of the connection between the base 110 and the modular component 200, 300. In at least one embodiment when additional notches are present, the modular component may be rotated relative to the based to engage the two notches that provide a preferred orientation for engagement of the modular humeral component by the modular component. In a further embodiment, a space will be defined by the notch 112 and the flange 212'/312' to receive an instrument to pry the mounted modular component 200, 300 from the baseplate 100.

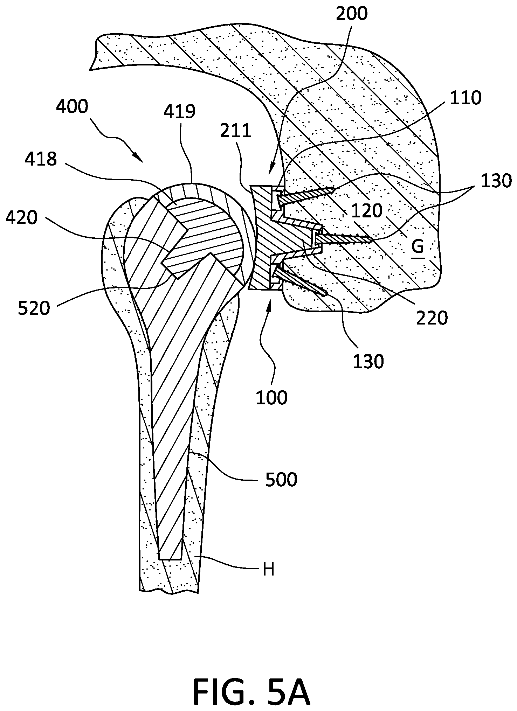

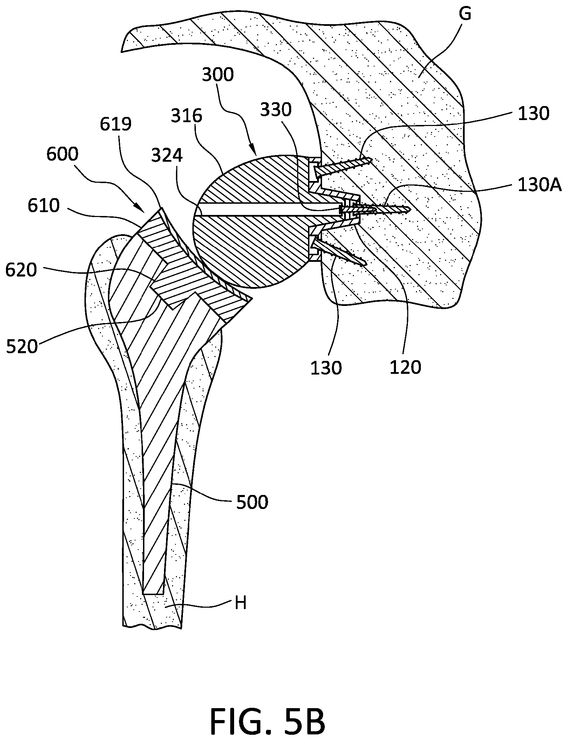

The illustrated base 110 includes five mounting holes 114A-114E with an axially centered hole 114A and four evenly spaced perimeter holes 114B-114E around the mounting surface 111. The holes 114A-114E are illustrated as having a shoulder 115 on which a screwhead, which is an example of an attachment mechanism 130, 130A, will make contact after insertion into the baseplate 100. Although five holes are illustrated, the number of holes could be reduced, including omission of the perimeter holes, or increased. In at least one embodiment, the central hole 114A defines a chamber 124 for receiving a modular component plug. In at least one embodiment, one or more variable angle locking screws 130, 130A are used to attach the baseplate 100 to the patient's glenoid bone G. FIGs. 5A and 5B illustrate the use of screws 130, 130A anchoring the baseplate 100 to the glenoid bone G. Examples of screw diameters include 4.5 mm to 5.0 mm and in a further example including the end points of that range. Although there are five holes illustrated, during a particular procedure, all five holes may not be utilized. In at least one embodiment, the flexibility in which holes 114A-114E to use and the variable angle locking screws 130, 130A provides flexibility to the orthopedic surgeon in securing the baseplate 100 to the patient's glenoid bone G. Examples of locking screw angles includes between 20 degrees and 30 degrees (with or without the end points) or perpendicular to the base 110. FIGs. 1B and 5B illustrate a screw 130A that includes a receiving cavity 134 for insertion of a torque limiting fastener inserted through the modular component.

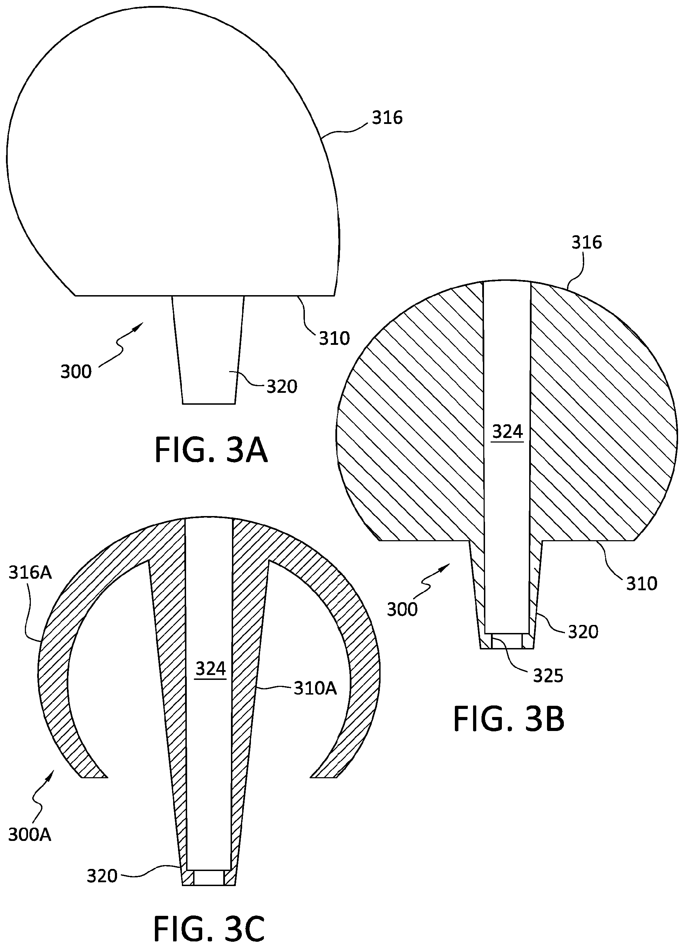

In at least one embodiment, the central axial opening 114A passes from the base 110 into and through the stem 120 to allow for the top of the locking screw 130A to be deeper into the baseplate 100 and to provide the chamber 124' for receiving plug, e.g., the Morse taper, of the modular component being mounted onto the baseplate 100. FIG. 1C illustrates the chamber 124' having receiving screw threads for engaging a torque limiting fastener 330'. As referenced above, the torque limiting fastener may engage locking screw 130A instead or in addition to other areas of the chamber 124'. FIG. 1C illustrates the modular component as the glenosphere component 300, 300' discussed in connection with FIGs. 3A-3F.

FIGs. 1D and 1E illustrate a pair of alternative baseplates 100A, 100B that have a partial wedge 116A and a full wedge 116B, respectively. One of ordinary skill in the art should appreciate that the presence of a wedge is potentially advantageous for addressing bone deformities of the glenoid while providing a secure attachment of the baseplate 100 to the patient's glenoid bone G. Alternatively, the baseplate 100 can have a 10 degree or 25 degree back as oppose to the neutral back illustrated in FIG. 1B.

FIGs. 2A (top view) and 2B (cross-section view) illustrate a modular glenoid component 200 having a concave surface 211 on a base 210 for receiving a prosthetic ball, glenosphere, or another convex shaped interface and a plug 220 extending from a surface 213 opposed to the concave surface 211 on a base 210. One of ordinary skill in the art should appreciate based on this disclosure, the concave surface can take a variety of shapes without departing from the modularity of the modular glenoid component 200. An example of a different shape is a variating surface with a non-uniform level of curvature over the concave surface. The plug 220 is configured to be inserted and frictionally engage the chamber 124 in the baseplate 100. In at least one embodiment, the plug 220 is a Morse taper central plug.

Although the modular glenoid component 200 is illustrated as being round in FIGs. 2A and 2C, the concave surface 211 may be elliptical, oval or other similar shapes when viewed from the top.

In a further embodiment illustrated in FIG. 2C, the modular glenoid component 200 includes a pair of flanges 212, 212 extending radially out from the anterior and posterior edges to provide additional surface area on which to apply leverage to remove the modular glenoid component 200 from the baseplate 100. In at least one embodiment, the flanges 212, 212 are aligned with the notches 112, 112 after the modular glenoid component 200 is implanted. In a further alternative embodiment, the flanges 212', 212' depend from the bottom surface 213 as illustrated in FIG. 2D. Flanges 212', 212' are shaped to be inserted into and engage at least a portion of a corresponding notch 112. In at least one further embodiment, the flange 212' and the notch 112 have an interference fit. As discussed above, the notch 112 and the flange 212' may define a space for receiving an instrument, or alternatively the flange 212' fully fills the space of the notch 112. In another alternative embodiment, the flanges 212, 212' illustrated in FIGs. 2C and 2D, respectively, are combined together.

In at least one embodiment, the modular glenoid component 200 is manufactured from Cobalt-Chromium (Co-Cr) to improve the life expectancy for the implant.

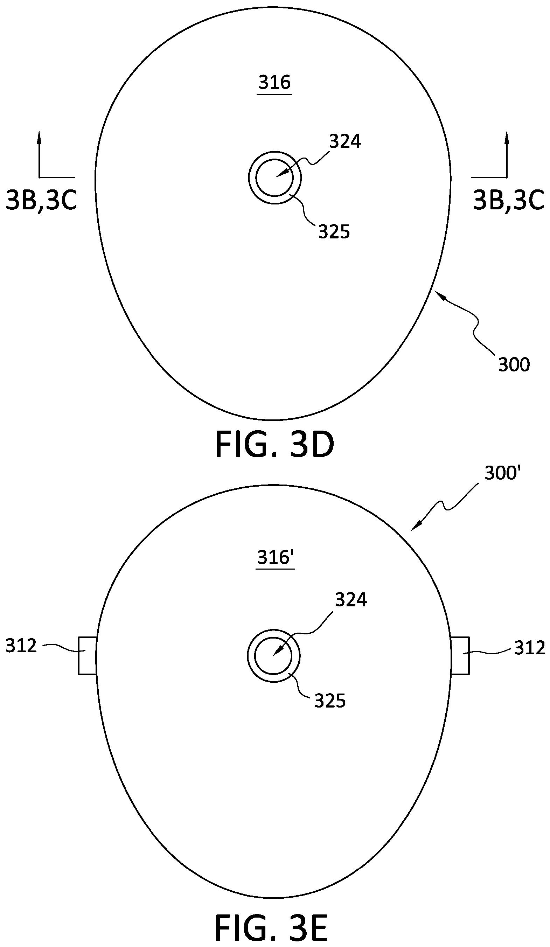

FIGs. 3A-3C illustrate a glenosphere component 300, 300A with a glenosphere 316 extending from a base 310 and a plug 320 extending from the base 310. In at least one embodiment, the plug 320 of the glenosphere component 300, 300A is similar to the plug 220 discussed above in connection with the modular glenoid component 200 including any further or alternative embodiments. In at least one embodiment, there is a 7-10 degree inferior tilt (*see*, *e.g*., FIGs. 3A, 3D, and 3E) added to the glenosphere that allows for improved glenosphere positioning for particular patient situations. The shape of the glenosphere may vary from that illustrated and the shape illustrated in FIGs. 3A, 3D, 3E and 5B are not intended to limit the exact shape as these are illustrative figures. More particularly, FIG. 3A illustrates a view taken anteriorly (i.e., standing in front of the shoulder when implanted).

FIGs. 3B-3D illustrate an embodiment that includes a passageway 324 passing through the axial center of the glenosphere 316, 316A and the plug 320. In at least one embodiment, the passageway 324 receives a fastener (not illustrated) to further secure the modular glenoid component 300 to the baseplate 100. In a further embodiment, the passageway includes a shoulder 325 on which a screwhead will make contact after insertion into the passageway 324.

FIGs. 3B and 3C illustrate alternative embodiments for the glenosphere that both illustrate the optional passageway 324. FIG. 3B illustrates a substantially solid glenosphere 316 other than the optional passageway 324. FIG. 3C illustrates a glenosphere 316A that is hollow other than an exterior surface and the base 310A that includes the plug 320 in this embodiment. The illustrated base 310A includes a support column on which the glenosphere 316A is supported.

In a further embodiment illustrated in FIG. 3E, the modular glenoid component 300' includes a pair of flanges 312, 312 extending radially out from the anterior and posterior edges of the glenosphere 316' to provide additional surface area on which to apply leverage to remove the modular glenoid component 300' from the baseplate 100. In a further alternative embodiment, the flanges 312', 312' depend from the glenosphere 316" when viewed from the top (or bottom) side as illustrated in FIG. 3F and similar to flanges 212', 212' discussed above in connection with the modular glenoid component 200 including alternative embodiments, hybrid flange embodiments, and engagement between flange and notch. Based on this disclosure, it should be understood that the flanges 312, 312' may extend from the glenosphere 316 or 316A.

In a further embodiment, the glenosphere may have a surface area having an arc extending for approximately 180 degrees to approximately 270 degrees. In a further embodiment, the glenosphere is approximately a three-quarters sphere and/or hemi-spherical.

In at least one embodiment, the glenosphere 316 is made from Co-Cr.

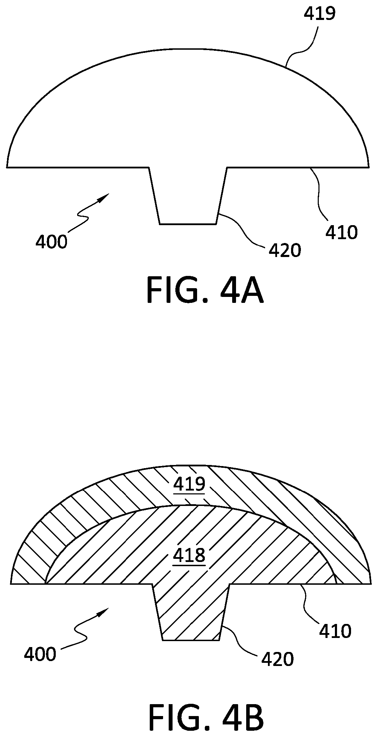

FIGs. 4A (side view) and 4B (cross-section view) illustrate a dual-mobility humeral head 400 for a ta-TSR. The illustrated humeral head 400 includes an outer shell 419 that is pressed over an inner head 418 of a base 410 and a plug 420 for insertion into a receiving cavity 520 of a humeral stem 500 embedded into the patient's humeral bone H as illustrated in FIG. 5A. The outer shell 419 is sized to interact with the concave surface 211 of the modular glenoid component 200 as illustrated in FIG. 5A. In at least one embodiment, the inner head 418 and the outer shell 419 are hemi-spherical domes. In at least one embodiment, the outer shell 419 is configured to slide relative to the inner hear 418. In a further embodiment, the outer shell 419 is smaller than an exterior surface of the inner head 418 to provide additional range of dual-mobility.

In at least one embodiment, the inner head 418 will be made from Co-Cr while the outer shell 419 will be made from high-density polyethylene, which will avoid the issue of having metal components rub against other metal components, which could lead to a faster wear on the components and potentially loose metal shavings within the shoulder socket. In an alternative embodiment, the outer shell 419 is removable from the inner head 418 to facilitate replacement, for example when the outer shell 419 is worn down from biomechanical movement about the shoulder joint.

In at least one embodiment, the humeral head 400 will have a centrally located plug 420 that is capable of being manually offset or is physically offset from the axial center to allow best coverage of the proximal humeral anatomic neck and metaphysis. The physical offset includes an eccentrically located plug 420, which when the modular humeral component is rotated provides different amounts of coverage to the humeral side.

FIG. 5A illustrates an example of how the modular humeral component, which is illustrated as the dual-mobility humeral head 400, cooperates with the modular component, which is illustrated as the modular glenoid component 200. The humeral head 400 is designed to fit into the concave surface 211 of the modular glenoid component 200, which for example is due to similar radiuses of curvature of both sides - the humeral head and the glenoid in a traditional TSR.

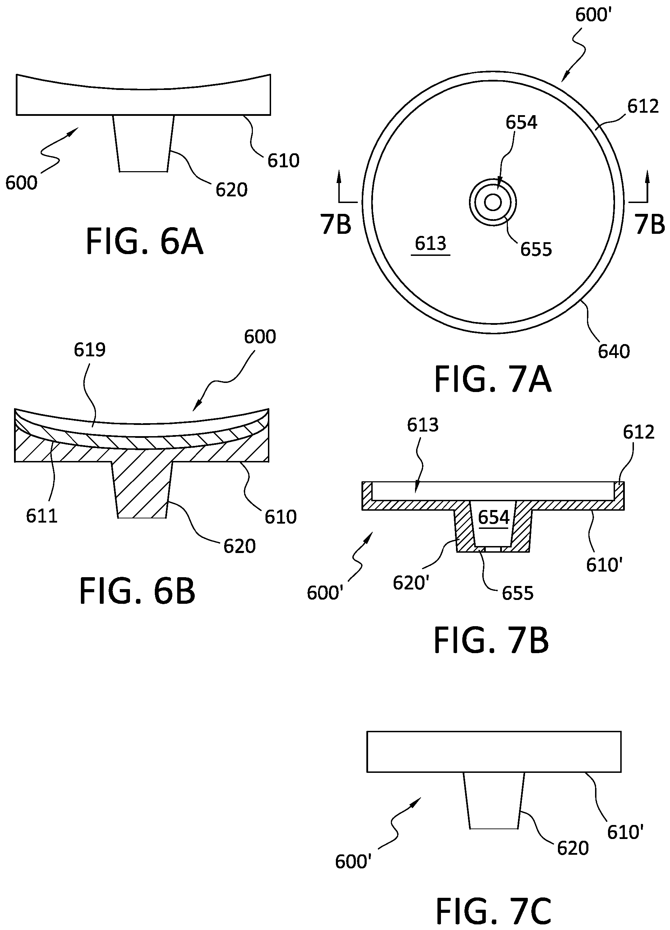

FIG. 5B illustrates an example of attachment of a humeral cup 600 as the modular humeral component to the humeral stem 500 while engaging with a glenosphere component 300 as the modular glenoid component to the baseplate 100. The humeral cup 600 is illustrated as having a shell 619 over a concave surface 611 (*see*, *e.g*., FIG. 6B) of the base 610 from which a plug 620 extends for engagement with the humeral stem 500. In an alternative embodiment, the glenosphere is approximately three-quarters spherical.

FIGs. 6A and 6B illustrate the humeral cup 600 for a r-TSR. The illustrated humeral cup 600 includes a concave surface (or receiving cavity) 611 on a base 610 and a plug 620 for insertion into the humeral stem 500 embedded into the patient's humeral bone H illustrated in FIGs. 5B, 7A and 7B. The concave surface 611 is adapted to interact with a glenosphere component 300. In an alternative embodiment, the concave surface 611 includes a variating surface with a non-uniform level of curvature over the concave surface. In at least one embodiment the humeral cup 600 will be made from Co-Cr, for example when the glenosphere component 300 has a high-density polyethylene cover, layer, and/or coating on it.

As illustrated in FIG. 6B, the humeral cup 600 may also have a core of Co-Cr or a similar metal to the humeral stem 500 (including a compatible metal) with a shell 619 over the concave surface 611 where the shell 619 is made from high-density polyethylene. In at least one embodiment, the polyethylene is seated into a metal concave surface 611. In an alternative embodiment, the concave surface 611 is substantially flat. In a further embodiment, the humeral cup 600' will have a shallow cylinder, for example with a height of 4 mm to 8 mm, a diameter of 30 mm, 35 mm or 40 mm to hold the various diameter humeral cup shells 619 that are inserted into a receiving cavity 613 defined by walls 612 that extend up from the base 610' as illustrated in FIG. 7B.

In at least one embodiment, the base 610 will have a centrally located plug 620 that is capable of being manually offset or is physically offset from the axial center to allow best coverage of the proximal humeral anatomic neck and metaphysis. The physical offset includes an eccentrically located plug 620, which when the modular humeral component is rotated provides different amounts of coverage to the humeral side.

FIGs. 7A-7C illustrate an example of an alternative humeral cup 600' that may be inserted into the humeral stem 500. In at least one embodiment, the plug 620' has a Morse taper to engage the humeral stem 500. The humeral cup 600' includes a mounting base 610' and a plug 620' extending from a bottom surface of the mounting base 610'. The humeral cup 600' includes a passageway 654 passing from the bottom of the shell receiving cavity (or chamber) 613 of the mounting base 610' through the plug 620' may receive a stem of the inserted shell in a further embodiment. In at least one embodiment, the receiving cavity 613 is short with the shell fitting into the receiving cavity 613, and in a further embodiment, the shell extends beyond the top of the receiving cavity 613 away from the mounting base 610'. The passageway 654 may include a shoulder 655 around an opening to facilitate the use of an attachment mechanism as illustrated in FIGs. 7A and 7B, which opening may be omitted as illustrated in FIG. 5B. In an alternative embodiment, the passageway 654 is added to the humeral cup 600 illustrated in FIGs. 6A and 6B. In at least one embodiment, the humeral cup 600' is made of the same or compatible material of the humeral stem 500.

In at least one embodiment, the shell is press-fit into the receiving cavity 611, 613. In at least one embodiment, the shell includes a curvature to it that has a uniform thickness, but in other embodiments the central portion is thicker than the edges. In a further embodiment, when the shell has been worn down, then it is removed and replaced. One approach for removing the shell is prying the polyethylene liner 619 from the receiving cavity 611, 613; another approach for removing the shell is to freeze it with liquid Nitrogen to freeze and shrink it before it pops out from the receiving cavity 611, 613.

In at least one embodiment, the stem of either humeral module is impacted onto the humeral stem such as a metaphyseal stem, short stem, or long stem through a variety of fixation techniques including, for example, a reverse Morse taper stem for the cavity 520 accepting the plug 420, 620 of the humeral head/cup 400, 600. Humeral stems known in the art with an adaptation for receiving the plug 420, 620 of the humeral head/cup 400/600 may be used. FIGs. 5A and 5B illustrate an example of a humeral stem 500 anchored in a humeral bone H.

In an alternative embodiment to the above-described modular components, modular glenoid components and modular humeral components (that collectively are examples of modular components) with a central passageway, the central passageway is omitted.

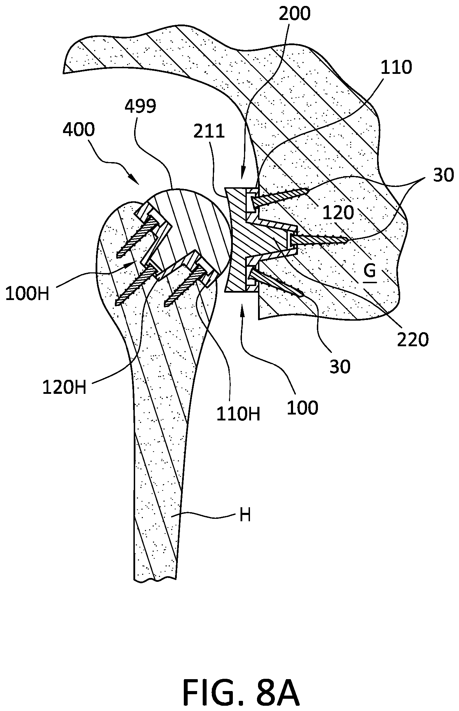

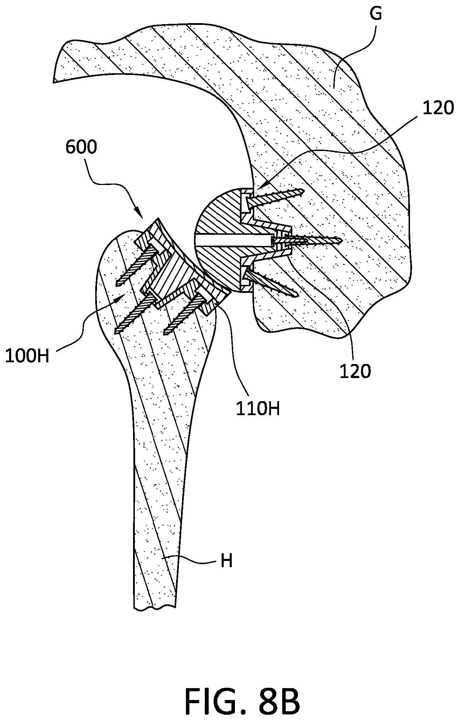

FIGs. 8A and 8B illustrate a configuration where the humeral stem 500 is replaced by a baseplate 100H, which in the illustrated embodiment is a second baseplate, being implanted in the humerus H. The baseplate 100H includes similar structures to the baseplate 100 including a base 110H with a mounting surface and notches and a stem 120H with a receiving cavity. In at least one embodiment, the humeral implanted baseplate 100H is identical to the glenoid implanted baseplate 100. In at least one further embodiment, this configuration allows for interchangeability of modular components between the two baseplates 100, 100H. In another embodiment, the humeral implanted baseplate 100H has a larger diameter for the base 110H and/or the stem 120H, a higher overall height of the base 110H and the stem 120H, a greater thickness of the base 110H, and/or a higher height of the stem 120H than the glenoid implanted baseplate 100. In at least one further embodiment, the modular components to be implanted in the humeral baseplate 100H would have corresponding adjustments. FIG. 8A illustrates an alternative humeral head 400 with an exterior surface 499 that is a one-piece construction for the humeral head 400 without any outer shell. Examples of diameters for the baseplates include about 25 mm, 29 mm, 33 mm, 37 mm, 41 mm, a range between 25 mm and 41 mm, or a range of 33 mm to 41 mm with or without the end points.

In at least one embodiment, the humeral stem and humeral baseplate are examples of a humeral base.

In a further embodiment to any of the embodiments discussed above having flanges that have an interference fit with notches, the humeral base and/or the baseplate may omitted a receiving cavity and instead rely on the interference fit between the flanges and the notches to secure the attached modular component when the system is implanted in a patient. Examples of the modular component include, for example, those discussed in this disclosure, a dual mobility humeral component, a humeral cup, a glenoid articular surface, and a glenosphere articular surface.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the root terms "include" and/or "have", when used in this specification, specify the presence of stated features, integers, steps, operations, elements, and/or components, but do not preclude the presence or addition of one or more other features, integers, steps, operations, elements, components, and/or groups thereof.

As used above "substantially," "generally," and other words of degree are relative modifiers intended to indicate permissible variation from the characteristic so modified. It is not intended to be limited to the absolute value or characteristic which it modifies but rather possessing more of the physical or functional characteristic than its opposite, and preferably, approaching or approximating such a physical or functional characteristic.

## Claims

1. A modular shoulder prosthesis system comprising:
a baseplate (100-100B) having
a base (110) with a plurality of attachment holes (114A-114B) passing therethrough and
a central stem (120) extending from said base (110) and axially centered with one of said plurality of attachment holes (114A);
a modular component (200, 300, 300A, 300') configured to be removably attached to said base (110), said modular component (200, 300, 300A, 300') having a plug (220, 320) for insertion into at least one attachment hole (114A) of said base (110), and
wherein said baseplate (100-100B) is capable of attachment to different modular components (200, 300, 300', 300A) to facilitate both traditional anatomic total shoulder replacement and reverse total shoulder replacement with a change in the modular component (200, 300, 300A, 300'); and
wherein the modular shoulder prosthesis system comprises a pair of notches (112) on opposed outer circumferential sides of said modular shoulder prosthesis system,
said notches (112) are configured to provide leverage points to facilitate separation of any attached modular component (200, 300, 300', 300A) from said baseplate (100-100B),
**characterized in that**
said pair of notches (112) are provided on opposed outer circumferential sides of said base (110),
said notches (112) extend down from a mounting surface (111) and in from said outer circumferential sides of said base (110), and said notches (112) are on anterior and posterior sides of said base (100).

2. The system according to claim 1, wherein
said modular component (200, 300, 300A, 300') includes a pair of flanges (212, 212', 312, 312') extending from opposing sides of said modular component (200, 300, 300A, 300'), said flanges (212, 212', 312, 312') configured to engage with an interference fit said notches (112) when said modular component (200, 300, 300A, 300') is attached to said base (110), and a length of said flange (212, 212', 312, 312') is less than or equal to a height of said notch, and said flanges configured to provide additional surface area on which to apply leverage for removal of said modular component (200, 300, 300A, 300') after installation onto said baseplate (100-100B); or
said modular component (200, 300, 300A, 300') includes a pair of flanges (212, 312) extending radially out from opposing anterior and posterior sides of said modular component (200, 300, 300A, 300'), said flanges (212, 312) configured to align with said notches (12) of said baseplate (100-100B) when said modular component (200, 300, 300A, 300') is attached to said base (110), and said flanges (212, 312) configured to provide additional surface area on which to apply leverage for removal of said modular component (200, 300, 300A, 300') after installation onto said baseplate (100-100B); or
said modular component (200, 300, 300A, 300') includes a pair of flanges (212', 312') extending down from opposing sides of said modular component (200, 300, 300A, 300'), said flanges (212', 312') configured to engage with an interference fit said notches (112) when said modular component (200, 300, 300A, 300') is attached to said base (110) and to provide a second leverage point on which to apply leverage for removal of said modular component (200, 300, 300', 300A) after installation onto said baseplate (100-100B).

3. The system according to claim 1 or 2, wherein said modular component (200, 300, 300A, 300') includes
a base (210) having a concave surface (211), and said plug (220) extends from a surface opposite (213) of said concave surface (211); or
a base (310, 310A), a glenosphere (316, 316', 316", 316A) extending at an angle from said base (300, 310A), and said plug (320) extends from a surface of said base opposite said glenosphere (316, 316', 316", 316A).

4. The system according to claim 3, wherein said glenosphere (316, 316', 316", 316A) is approximately a three-quarters sphere.

5. The system according to any one of claims 1-4, further comprising:
a humeral base (100H, 500) that is either a humeral stem (500) or a second baseplate (100H), said humeral base (100H, 500) having a receiving cavity (520) extending in from one face, optionally said humeral base (100H, 500) includes a pair of notches (112) on opposed outer circumferential sides of said humeral base (100H, 500), said pair of notches (112) extend down from a mounting surface and in from said outer circumferential sides of said humeral base (100H, 500); and
a modular humeral component (400, 600, 600') configured to cooperate with said modular component (200, 300, 300', 300A), said modular humeral component (400, 600, 600') having a plug (420, 620) configured for removable insertion into the receiving cavity (520) of said humeral base (110H, 500), and
wherein said humeral base (100H, 500) is capable of attachment to different modular humeral components (400, 600, 600') to facilitate both traditional anatomic total shoulder replacement and reverse total shoulder replacement with a change in the modular component (200, 300, 300', 300A) and change in the modular humeral component (400, 600, 600').

6. The system according to any one of claims 1-4, further comprising:
a second baseplate (100H) configured to attach to a humerus (H),
said second baseplate (100H) having
a base (110H) with at least two notches (112) spaced around an outer circumferential edge of said base (110H), said notches (112) extend down from a mounting surface and in from said outer circumferential sides of said base (110H), and
a central stem (120H) extending from said base (110H) and axially centered and having a receiving cavity;
a modular humeral component (400, 600, 600') configured to cooperate with said modular component (200, 300, 300', 300A), said modular humeral component (400, 600, 600') having a plug (420, 620, 620') configured for removable insertion into the receiving cavity of said second baseplate (110H), and
wherein said first baseplate (100-100B) is configured to attach to a glenoid (G) and said second baseplate (100H) is capable of attachment to different modular humeral components (400, 600, 600') to facilitate both traditional anatomic total shoulder replacement and reverse total shoulder replacement with a change in the modular component (200, 300, 300', 300A) and change in the modular humeral component (400, 600, 600'); and
said notches (112) of said second baseplate (100H) are configured to provide leverage points to facilitate separation of any attached modular humeral component (400, 600, 600') from said second baseplate (100H).

7. The system according to claim 6, wherein said second baseplate (110H) is larger than said first baseplate (100-100B) in terms of diameter, thickness, and/or height.

8. The system according to any one of claims 5-7, wherein each baseplate (100-100B, 110H) includes three or more notches, said modular component (200, 300, 300', 300A) and said modular humeral component (400, 600, 600') are each configured to have said two flanges attach to two notches of the respective baseplate (100-100B, 110H) when implanted to provide a selectable orientation relative to the other of said modular component (200, 300, 300', 300A) and said modular humeral component (400, 600, 600').

9. The system according to any one of claims 6-8, wherein
said modular humeral component (400, 600, 600') includes a pair of flanges extending from opposing sides of said modular humeral component (400, 600, 600'), said flanges configured to engage with an interference fit said notches of said second baseplate (100H, 500) when said modular humeral component (400, 600, 600') is attached to said second baseplate (100H, 500), said flanges of said modular humeral component (400, 600, 600') configured to provide additional surface area on which to apply leverage for removal of said modular humeral component (400, 600, 600') after installation onto said second baseplate (100H, 500); and/or
said post (420, 620, 620') of said modular humeral component (400, 600, 600') is eccentrically offset or manually offsetable from an axial center of said modular humeral component (400, 600, 600'); and/or
said modular component (200, 300, 300', 300A) and said modular humeral component (400, 600, 600') are interchangeable with each other.

10. The system according to any of claims 5-9, wherein said modular humeral component (600, 600') further includes
a base (610, 610') having a receiving cavity (611, 613),
said plug (620, 620') extends from a surface opposite of said receiving cavity (611, 613), and
a shell (619) inserted into said receiving cavity (611, 613).

11. The system according to any one of claims 5-9, wherein said modular humeral component (400) includes
a base (410) having an inner head (418);
an outer shell (419) over said inner head (418); and
said plug (420) extending from said base (410) on a surface opposing said inner head (418).

12. The system according to claim 11, wherein said inner head (418) is a hemi-spherical dome and said outer shell (419) is a hemi-spherical cap that fits over said inner head (418),
said hemi-spherical cap (419) is configured to slide relative about said hemi-spherical dome (418) to allow for dual mobility between said hemi-spherical cap (419) and said hemi-spherical dome (418).

13. The system according to claim 11 or 12, wherein
said inner head (418) includes Cobalt-Chromium and said outer shell (419) includes a high-density polyethylene; and/or
said inner head (418) of said base (410) and said outer shell (419) are configured to slide relative to each other to provide dual mobility.

14. The system according to any one of claims 1-13, further comprising a plurality of attachment mechanisms (30), wherein the attachment holes (114B-114E) of said base of said baseplate (100-100B, 100H) are configured to engage with said attachment mechanisms (30).

15. The system according to any one of claims 1-14, wherein said baseplate (100-100B) includes a mounting surface (111) that is substantially planar.

## Patentansprüche

1. Modulares Schulterprothesensystem, aufweisend:
eine Basisplatte (100-100B) aufweisend
eine Basis (110) mit einer Mehrzahl von Befestigungslöchern (114A-114B), die durch diese hindurchgehen, und
einen zentralen Schaft (120), der sich von der Basis (110) aus erstreckt und axial mit einem aus der Mehrzahl von Befestigungslöchern (114A) zentriert ist,
eine modulare Komponente (200, 300, 300A, 300'), die konfiguriert ist, um lösbar an der Basis (110) befestigt zu sein, wobei die modulare Komponente (200, 300, 300A, 300') einen Zapfen (220, 320) zum Einsetzen in mindestens ein Befestigungsloch (114A) der Basis (110) aufweist, und
wobei die Basisplatte (100-100B) für eine Befestigung an verschiedenen modularen Komponenten (200, 300, 300', 300A) geeignet ist, um sowohl einen traditionellen anatomischen Schulter-Totalersatz als auch einen inversen Schulter-Totalersatz mit einem Wechsel der modularen Komponente (200, 300, 300A, 300') zu ermöglichen, und
wobei das modulare Schulterprothesensystem ein Paar Einkerbungen (112) an entgegengesetzten Außenumfangsseiten des modularen Schulterprothesensystems aufweist,
wobei die Einkerbungen (112) konfiguriert sind, um Hebelpunkte bereitzustellen, um ein Trennen irgendeiner befestigten modularen Komponente (200, 300, 300', 300A) von der Basisplatte (100-100B) zu erleichtern,
**dadurch gekennzeichnet, dass**
das Paar von Einkerbungen (112) an entgegengesetzten Außenumfangsseiten der Basis (110) vorgesehen ist,
die Einkerbungen (112) sich von einer Montagefläche (111) nach unten und von den Außenumfangsseiten der Basis (110) nach innen erstrecken, und
die Einkerbungen (112) an einer anterioren und einer posterioren Seite der Basis (100) sind.

2. System gemäß Anspruch 1, wobei
die modulare Komponente (200, 300, 300A, 300') ein Paar Flansche (212, 212', 312, 312') aufweist, die sich von entgegengesetzten Seiten der modularen Komponente (200, 300, 300A, 300') erstrecken, wobei die Flansche (212, 212', 312, 312') konfiguriert sind, um mit den Einkerbungen (112) in Presspassung in Eingriff zu gelangen, wenn die modulare Komponente (200, 300, 300A, 300') an der Basis (110) befestigt ist, und eine Länge des Flansches (212, 212', 312, 312') kleiner oder gleich einer Höhe der Einkerbung ist, und wobei die Flansche konfiguriert sind, um eine zusätzliche Fläche bereitzustellen, auf die zum Entfernen der modularen Komponente (200, 300, 300A, 300') nach dem Installieren an der Basisplatte (100-100B) Hebelkraft ausgeübt werden kann, oder
die modulare Komponente (200, 300, 300A, 300') ein Paar Flansche (212, 312) aufweist, die sich radial von einer einander entgegengesetzten anterioren und posterioren Seite der modularen Komponente (200, 300, 300A, 300') aus erstrecken, wobei die Flansche (212, 312) konfiguriert sind, um zu den Einkerbungen (12) der Basisplatte (100-100B) ausgerichtet zu sein, wenn die modulare Komponente (200, 300, 300A, 300') an der Basis (110) befestigt ist, und wobei die Flansche (212, 312) konfiguriert sind, um eine zusätzliche Fläche bereitzustellen, auf die zum Entfernen der modularen Komponente (200, 300, 300A, 300') nach dem Installieren an der Basisplatte (100-100B) Hebelkraft ausgeübt werden kann, oder
die modulare Komponente (200, 300, 300A, 300') ein Paar Flansche (212', 312') aufweist, die sich von entgegengesetzten Seiten der modularen Komponente (200, 300, 300A, 300') nach unten erstrecken, wobei die Flansche (212', 312') konfiguriert sind, um mit den Einkerbungen (112) in Presspassung in Eingriff zu gelangen, wenn die modulare Komponente (200, 300, 300A, 300') an der Basisplatte (110) befestigt ist, und um einen zweiten Hebelpunkt bereitzustellen, an dem eine Hebelkraft zum Entfernen der modularen Komponente (200, 300, 300', 300A) nach dem Installieren an der Basisplatte (100-100B) ausgeübt werden kann.

3. System gemäß Anspruch 1 oder 2, wobei die modulare Komponente (200, 300, 300A, 300') aufweist:
eine Basis (210) aufweisend eine konkave Fläche (211) und wobei sich der Zapfen (220) von einer zu der konkaven Fläche (211) entgegengesetzten Fläche (213) aus erstreckt, oder
eine Basis (310, 310A), eine Glenosphäre (316, 316', 316'' 316A), die sich in einem Winkel von der Basis (300, 310A) aus erstreckt, und wobei sich der Zapfen (320) von einer zu der Glenosphäre (316, 316', 316" 316A) entgegengesetzten Fläche der Basis erstreckt.

4. System gemäß Anspruch 3, wobei die Glenosphäre (316, 316', 316", 316A) annähernd eine Dreiviertelkugel ist.

5. System gemäß irgendeinem der Ansprüche 1-4, ferner aufweisend:
eine Humerusbasis (100H, 500), die entweder ein Humerusschaft (500) oder eine zweite Basisplatte (100H) ist, wobei die Humerusbasis (100H, 500) einen Aufnahmeraum (520) hat, der sich von einer Fläche aus nach innen erstreckt, wobei die Humerusbasis (100H, 500) optional ein Paar Einkerbungen (112) an entgegengesetzten Außenumfangsseiten der Humerusbasis (100H, 500) aufweist, wobei sich das Paar von Einkerbungen (112) von einer Montagefläche aus nach unten und von den Außenumfangsseiten der Humerusbasis (100H, 500) aus nach innen erstreckt, und
eine modulare Humeruskomponente (400, 600, 600'), die konfiguriert ist, um mit der modularen Komponente (200, 300, 300', 300A) zusammenzuwirken, wobei die modulare Humeruskomponente (400, 600, 600') einen Zapfen (420, 620) hat, der zum lösbaren Einsetzen in den Aufnahmeraum (520) der Humerusbasis (110H, 500) konfiguriert ist, und
wobei die Humerusbasis (100H, 500) für ein Befestigen an verschiedenen modularen Humeruskomponenten (400, 600, 600') geeignet ist, um sowohl den traditionellen anatomischen Schulter-Totalersatz als auch den inversen Total-Schulterersatz mit einem Wechsel der modularen Komponente (200, 300, 300', 300A) und einem Wechsel der modularen Humeruskomponente (400, 600, 600') zu ermöglichen.

6. System gemäß irgendeinem der Ansprüche 1-4, ferner aufweisend:
eine zweite Basisplatte (100H), die für ein Befestigen an einem Humerus (H) konfiguriert ist,
wobei die zweite Basisplatte (100H) aufweist:
eine Basis (110H) mit mindestens zwei Einkerbungen (112), die um einen Außenumfangsrand der Basis (110H) herum beabstandet sind, wobei sich die Einkerbungen (112) von einer Montagefläche aus nach unten und von den Außenumfangsseiten der Basis (110H) aus nach innen erstrecken, und
einen zentralen Schaft (120H), der sich von der Basis (110H) aus erstreckt, und axial zentriert ist und einen Aufnahmeraum hat,
eine modulare Humeruskomponente (400, 600, 600'), die konfiguriert ist, um mit der modularen Komponente (200, 300, 300', 300A) zusammenzuwirken, wobei die modulare Humeruskomponente (400, 600, 600') einen Zapfen (420, 620, 620') hat, der zum lösbaren Einsetzen in den Aufnahmeraum der zweiten Basisplatte (110H) konfiguriert ist, und
wobei die erste Basisplatte (100-100B) konfiguriert ist, um an einem Glenoid (G) befestigt zu sein und die zweite Basisplatte (100H) an verschiedenen modularen Humeruskomponenten (400, 600, 600') befestigt sein kann, um sowohl den traditionellen anatomischen Schulter-Totalersatz als auch den inversen Schulter-Totalersatz mit einem Wechsel der modularen Komponente (200, 300, 300', 300A) und einem Wechsel der modularen Humeruskomponente (400, 600, 600') zu ermöglichen, und
die Einkerbungen (112) der zweiten Basisplatte (100H) konfiguriert sind, um Hebelpunkte bereitzustellen, um ein Trennen irgendeiner befestigten modularen Humeruskomponente (400, 600, 600') von der zweiten Basisplatte (100H) zu erleichtern.

7. System gemäß Anspruch 6, wobei die zweite Basisplatte (110H) hinsichtlich Durchmesser, Dicke und/oder Höhe größer als die erste Basisplatte (100-100B) ist.

8. System gemäß irgendeinem der Ansprüche 5-7, wobei jede Basisplatte (100-100B, 110H) drei oder mehr Einkerbungen aufweist, wobei die modulare Komponente (200, 300, 300', 300A) und die modulare Humeruskomponente (400, 600, 600') jeweils konfiguriert sind, damit die zwei Flansche beim Implantieren an zwei Einkerbungen der jeweiligen Basisplatte (100-100B, 110H) befestigt sein können, um eine wählbare Ausrichtung relativ zu der anderen der modularen Komponente (200, 300, 300', 300A) und der modularen Humeruskomponente (400, 600, 600') bereitzustellen.

9. System gemäß irgendeinem der Ansprüche 6-8, wobei
die modulare Humeruskomponente (400, 600, 600') ein Paar von Flanschen aufweist, die sich von entgegengesetzten Seiten der modularen Humeruskomponente (400, 600, 600') aus erstrecken, wobei die Flansche konfiguriert sind, um mit den Einkerbungen der zweiten Basisplatte (100H, 500) in Presspassung in Eingriff zu gelangen, wenn die modulare Humeruskomponente (400, 600, 600') an der zweiten Basisplatte (100H, 500) angebracht ist, wobei die Flansche der modularen Humeruskomponente (400, 600, 600') konfiguriert sind, um eine zusätzliche Fläche bereitzustellen, auf die zum Entfernen der modularen Humeruskomponente (400, 600, 600') nach dem Installieren an der zweiten Basisplatte (100H, 500) Hebelkraft ausgeübt werden kann, und/oder
der Schaft (420, 620, 620') der modularen Humeruskomponente (400, 600, 600') exzentrisch versetzt oder manuell versetzbar gegenüber einer axialen Mitte der modularen Humeruskomponente (400, 600, 600') ist, und/oder
die modulare Komponente (200, 300, 300', 300A) und die modulare Humeruskomponente (400, 600, 600') untereinander austauschbar sind.

10. System gemäß irgendeinem der Ansprüche 5-9, wobei die modulare Humeruskomponente (600, 600') ferner aufweist
eine Basis (610, 610'), die einen Aufnahmeraum (611, 613) aufweist,
wobei sich der Zapfen (620, 620') von einer Fläche aus gegenüber dem Aufnahmeraum (611, 613) erstreckt, und
eine in den Aufnahmeraum (611, 613) eingesetzte Hülse (619).

11. System gemäß irgendeinem der Ansprüche 5-9, wobei die modulare Humeruskomponente (400) aufweist
eine Basis (410), die einen Innenkopf (418) aufweist,
eine Außenhülse (419) über dem Innenkopf (418), und
den Zapfen (420), der sich von der Basis (410) aus auf einer der dem Innenkopf gegenüberliegenden Fläche (418) erstreckt.

12. System gemäß Anspruch 11, wobei der Innenkopf (418) eine halbkugelförmige Kuppel ist und die Außenhülse (419) eine halbkugelförmige Kappe ist, die über den Innenkopf (418) passt,
wobei die halbkugelförmige Kappe (419) konfiguriert ist, um relativ um die halbkugelförmigen Kuppel (418) zu gleiten, um eine doppelte Bewegbarkeit zwischen der halbkugelförmigen Kappe (419) und der halbkugelförmigen Kuppel (418) zu ermöglichen.

13. System gemäß Anspruch 11 oder 12, wobei
der Innenkopf (418) Kobalt-Chrom aufweist und die Außenhülse (419) hochdichtes Polyethylen aufweist, und/oder
der Innenkopf (418) der Basis (410) und die Außenhülse (419) konfiguriert sind, um relativ zueinander zu gleiten, um eine doppelte Bewegbarkeit zu gewährleisten.

14. System gemäß irgendeinem der Ansprüche 1-13, ferner aufweisend eine Mehrzahl von Befestigungsmechanismen (30), wobei die Befestigungslöcher (114B-114E) der Basis der Basisplatte (100-100B, 100H) konfiguriert sind, um mit den Befestigungsmechanismen (30) in Eingriff zu gelangen.

15. System gemäß irgendeinem der Ansprüche 1-14, wobei die Basisplatte (100-100B) eine Montagefläche (111) aufweist, die im Wesentlichen planar ist.

## Revendications

1. Système de prothèse d'épaule modulaire, comprenant :
une plaque de base (100-100B) comportant
une base (110) avec une pluralité de trous de fixation (114A-114B) passant à travers celle-ci ; et
une tige centrale (120) s'étendant à partir de ladite base (110) et centrée axialement avec l'un parmi la pluralité de trous de fixation (114A) ;
un composant modulaire (200, 300, 300A, 300') configuré pour être fixé de manière amovible à ladite base (110), ledit composant modulaire (200, 300, 300A, 300') comportant un bouchon (220, 320) destiné à être inséré dans au moins un trou de fixation (114A) de ladite base (110), et
dans lequel ladite plaque de base (100-100B) peut être fixée à différents composants modulaires (200, 300, 300', 300A) afin de faciliter à la fois un remplacement total anatomique d'épaule traditionnel et un remplacement total d'épaule inversé, en changeant de composant modulaire (200, 300, 300A, 300') ; et
dans lequel le système de prothèse d'épaule modulaire comprend une paire d'encoches (112) situées sur des côtés circonférentiels extérieurs opposés dudit système de prothèse d'épaule modulaire,
lesdites encoches (112) sont configurées pour fournir des points de levier pour faciliter la séparation de tout composant modulaire fixé (200, 300, 300', 300A) de ladite plaque de base (100-100B),
**caractérisé en ce que**
ladite paire d'encoches (112) est prévue sur des côtés circonférentiels extérieurs opposés de ladite base (110),
lesdites encoches (112) s'étendent vers le bas à partir d'une surface de montage (111) et vers l'intérieur à partir desdits côtés circonférentiels extérieurs de ladite base (110), et
lesdites encoches (112) se trouvent sur des côtés antérieur et postérieur de ladite base (100).

2. Système selon la revendication 1, dans lequel
ledit composant modulaire (200, 300, 300A, 300') comprend une paire de brides (212, 212', 312, 312') s'étendant à partir de côtés opposés dudit composant modulaire (200, 300, 300A, 300'), lesdites brides (212, 212', 312, 312') étant configurées pour venir en prise par ajustement serré dans lesdites encoches (112) lorsque ledit composant modulaire (200, 300, 300A, 300') est fixé à ladite base (110), et une longueur de ladite bride (212, 212', 312, 312') est inférieure ou égale à une hauteur de ladite encoche, et lesdites brides sont configurées pour offrir une surface supplémentaire sur laquelle exercer un effet de levier en vue du retrait dudit composant modulaire (200, 300, 300A, 300') après son installation sur ladite plaque de base (100-100B) ; ou
ledit composant modulaire (200, 300, 300A, 300') comprend une paire de brides (212, 312) s'étendant radialement vers l'extérieur à partir de côtés antérieur et postérieur opposés dudit composant modulaire (200, 300, 300A, 300'), lesdites brides (212, 312) étant configurées pour s'aligner avec lesdites encoches (12) de ladite plaque de base (100-100B) lorsque ledit composant modulaire (200, 300, 300A, 300') est fixé à ladite base (110), et lesdites brides (212, 312) étant configurées pour offrir une surface supplémentaire sur laquelle exercer un effet de levier en vue du retrait dudit composant modulaire (200, 300, 300A, 300') après son installation sur ladite plaque de base (100-100B) ; ou
ledit composant modulaire (200, 300, 300A, 300') comprend une paire de brides (212', 312') s'étendant vers le bas à partir de côtés opposés dudit composant modulaire (200, 300, 300A, 300'), lesdites brides (212', 312') étant configurées pour venir en prise par ajustement serré dans lesdites encoches (112) lorsque ledit composant modulaire (200, 300, 300A, 300') est fixé à ladite embase (110) et pour fournir un deuxième point de levier sur lequel exercer une force de levier en vue du retrait dudit composant modulaire (200, 300, 300', 300A) après son installation sur ladite plaque de base (100-100B).

3. Système selon la revendication 1 ou 2, dans lequel ledit composant modulaire (200, 300, 300A, 300') comprend
une base (210) présentant une surface concave (211), et ledit bouchon (220) s'étend à partir d'une surface opposée (213) de ladite surface concave (211) ; ou
une base (310, 310A), une glénosphère (316, 316', 316", 316A) s'étendant selon un angle à partir de ladite base (300, 310A), et ledit bouchon (320) s'étendant à partir d'une surface de ladite base opposée à ladite glénosphère (316, 316', 316", 316A).

4. Système selon la revendication 3, dans lequel ladite glénosphère (316, 316', 316", 316A) est approximativement une sphère aux trois quarts.

5. Système selon l'une quelconque des revendications 1 à 4, comprenant en outre :
une base humérale (100H, 500) qui est soit une tige humérale (500), soit une deuxième plaque de base (100H), ladite base humérale (100H, 500) comportant une cavité de réception (520) s'étendant vers l'intérieur à partir d'une face, ladite base humérale (100H, 500) comprenant en option une paire d'encoches (112) sur des côtés circonférentiels extérieurs opposés de ladite base humérale (100H, 500), ladite paire d'encoches (112) s'étendant vers le bas à partir d'une surface de montage et vers l'intérieur à partir desdits côtés circonférentiels extérieurs de ladite base humérale (100H, 500) ; et
un composant huméral modulaire (400, 600, 600') configuré pour coopérer avec ledit composant modulaire (200, 300, 300', 300A), ledit composant huméral modulaire (400, 600, 600') comportant un bouchon (420, 620) configuré pour être inséré de manière amovible dans la cavité de réception (520) de ladite base humérale (110H, 500), et
dans lequel ladite base humérale (100H, 500) peut être fixée à différents composants huméraux modulaires (400, 600, 600') afin de faciliter à la fois un remplacement total anatomique d'épaule traditionnel et un remplacement total d'épaule inversé, en changeant le composant modulaire (200, 300, 300', 300A) et en changeant le composant huméral modulaire (400, 600, 600').

6. Système selon l'une quelconque des revendications 1 à 4, comprenant en outre :
une deuxième plaque de base (100H) configurée pour se fixer à un humérus (H),
ladite deuxième plaque de base (100H) comportant
une base (110H) avec au moins deux encoches (112) espacées autour d'un bord circonférentiel extérieur de ladite base (110H), lesdites encoches (112) s'étendant vers le bas à partir d'une surface de montage et vers l'intérieur à partir desdits côtés circonférentiels extérieurs de ladite base (110H), et
une tige centrale (120H) s'étendant à partir de ladite base (110H) et centrée axialement et comportant une cavité de réception ;
un composant huméral modulaire (400, 600, 600') configuré pour coopérer avec ledit composant modulaire (200, 300, 300', 300A), ledit composant huméral modulaire (400, 600, 600') comportant un bouchon (420, 620, 620') configuré pour être inséré de manière amovible dans la cavité de réception de ladite deuxième plaque de base (110H), et
dans lequel ladite première plaque de base (100-100B) est configurée pour se fixer à une glène (G) et ladite deuxième plaque de base (100H) peut se fixer à différents composants huméraux modulaires (400, 600, 600') afin de faciliter à la fois un remplacement total anatomique d'épaule traditionnel et un remplacement total d'épaule inversé, en changeant le composant modulaire (200, 300, 300', 300A) et en changeant le composant huméral modulaire (400, 600, 600') ; et
lesdites encoches (112) de ladite deuxième plaque de base (100H) sont configurées pour fournir des points de levier permettant de faciliter la séparation de tout composant huméral modulaire fixé (400, 600, 600') de ladite deuxième plaque de base (100H).

7. Système selon la revendication 6, dans lequel ladite deuxième plaque de base (110H) est plus grande que ladite première plaque de base (100-100B) en termes de diamètre, d'épaisseur et/ou de hauteur.

8. Système selon l'une quelconque des revendications 5 à 7, dans lequel chaque plaque de base (100-100B, 110H) comprend au trois encoches ou plus, ledit composant modulaire (200, 300, 300', 300A) et ledit composant huméral modulaire (400, 600, 600') sont chacun configurés de manière à ce que lesdites deux brides s'attachent à deux encoches de la plaque de base respective (100-100B, 110H) une fois implantés, afin d'assurer une orientation sélectionnable par rapport à l'autre parmi ledit composant modulaire (200, 300, 300', 300A) et ledit composant huméral modulaire (400, 600, 600').

9. Système selon l'une quelconque des revendications 6 à 8, dans lequel
ledit composant huméral modulaire (400, 600, 600') comprend une paire de brides s'étendant à partir de côtés opposés dudit composant huméral modulaire (400, 600, 600'), lesdites brides étant configurées pour venir en prise par ajustement serré dans lesdites encoches de ladite deuxième plaque de base (100H, 500) lorsque ledit composant huméral modulaire (400, 600, 600') est fixé à ladite deuxième plaque de base (100H, 500), lesdites brides dudit composant huméral modulaire (400, 600, 600') étant configurées pour offrir une surface supplémentaire sur laquelle exercer un effet de levier en vue du retrait dudit composant huméral modulaire (400, 600, 600') après son installation sur ladite deuxième plaque de base (100H, 500) ; et/ou
ledit montant (420, 620, 620') dudit composant huméral modulaire (400, 600, 600') est décalé de manière excentrique ou peut être décalé manuellement par rapport à un centre axial dudit composant huméral modulaire (400, 600, 600') ; et/ou
ledit composant modulaire (200, 300, 300', 300A) et ledit composant huméral modulaire (400, 600, 600') sont interchangeables entre eux.

10. Système selon l'une quelconque des revendications 5 à 9, dans lequel ledit composant huméral modulaire (600, 600') comprend en outre
une base (610, 610') comportant une cavité de réception (611, 613),
ledit bouchon (620, 620') s'étend à partir d'une surface opposée à ladite cavité de réception (611, 613), et
une coque (619) insérée dans ladite cavité de réception (611, 613).

11. Système selon l'une quelconque des revendications 5 à 9, dans lequel ledit composant huméral modulaire (400) comprend
une base (410) comportant une tête interne (418) ;
une coque externe (419) sur ladite tête interne (418) ; et
ledit bouchon (420) s'étendant depuis ladite base (410) sur une surface opposée à ladite tête interne (418).

12. Système selon la revendication 11, dans lequel ladite tête interne (418) est un dôme hémisphérique et ladite coque externe (419) est un capuchon hémisphérique qui s'adapte sur ladite tête interne (418),
ledit capuchon hémisphérique (419) est configuré pour coulisser par rapport autour dudit dôme hémisphérique (418) afin de permettre une double mobilité entre ledit capuchon hémisphérique (419) et ledit dôme hémisphérique (418).

13. Système selon la revendication 11 ou 12, dans lequel
ladite tête interne (418) comprend du cobalt-chrome et ladite coque externe (419) comprend du polyéthylène haute densité ; et/ou
ladite tête interne (418) de ladite base (410) et ladite coque externe (419) sont configurées pour coulisser l'une par rapport à l'autre afin d'assurer une double mobilité.

14. Système selon l'une quelconque des revendications 1 à 13, comprenant en outre une pluralité de mécanismes de fixation (30), dans lequel les trous de fixation (114B-114E) de ladite base de ladite plaque de base (100-100B, 100H) sont configurés pour venir en prise avec lesdits mécanismes de fixation (30).

15. Système selon l'une quelconque des revendications 1 à 14, dans lequel ladite plaque de base (100-100B) comprend une surface de montage (111) qui est sensiblement plane.
